# EUROPÄISCHE PATENTSCHRIFT

(11) **EP 3 622 058 B1**
(45) Veröffentlichungstag und Bekanntmachung des Hinweises auf die Patenterteilung: **09.11.2022**
(21) Anmeldenummer: 18724121.1
(22) Anmeldetag: 17.04.2018
(51) Int. Cl.: C12Q 1/58, C12N 9/00

(54) **PYRUVATCARBOXYLASE**
PYRUVATE CARBOXYLASE
PYRUVATE CARBOXYLASE

(30) Priorität: 11.05.2017 DE 102017004566
(43) Veröffentlichungstag der Anmeldung: 18.03.2020
(73) Patentinhaber: Forschungszentrum Jülich GmbH, 52425 Jülich (DE)
(72) Erfinder: KORTMANN, Maike, 52066 Aachen (DE); BAUMGART, Meike, 50259 Pulheim (DE); BOTT, Michael, 52428 Jülich (DE)
(86) Internationale Anmeldenummer: PCT/DE2018/000108
(87) Internationale Veröffentlichungsnummer: WO 2018/206024

(56) Entgegenhaltungen:
- EP-A2- 1 108 790
- WO-A2-02/31158
- US-B2- 9 644 226
- OHNISHI J ET AL: "A novel methodology employing Corynebacterium glutamicum genome information to generate a new L-lysine-producing mutant", APPLIED MICROBIOLOGY AND BIOTECHNOLOGY, SPRINGER, DE, Bd. 58, Nr. 2, 1. Februar 2002 (2002-02-01), Seiten 217-223, XP002289984, ISSN: 0175-7598, DOI: 10.1007/S00253-001-0883-6

## Beschreibung

Die Erfindung betrifft eine Pyruvatcarboxylase und eine für die Pyruvatcarboxylase kodierende DNA, ein Plasmid enthaltend die DNA sowie einen Mikroorganismus zur Produktion und ein Verfahren zur Herstellung von Produkten, deren Biosynthese Oxalacetat als Vorstufe beinhalten, ein Chromosom und ein Screeningverfahren.

*Corynebacterium glutamicum* wird industriell für die Herstellung von Aminosäuren, insbesondere L-Glutamat und L-Lysin, genutzt. Für die Produktion von L-Lysin wird dem Citrat-Zyklus das Intermediat Oxalacetat entzogen, da es als Vorstufe für Aminosäuren oder Salze der Aminosäuren der Aspartatfamilie dient. Diese sind L-Aspartat, L-Asparagin, L-Lysin, L-Methionin, L-Threonin und L-Isoleucin. Damit der Citrat-Zyklus trotz des Entzugs dieser Intermediate weiter ablaufen kann, muss er durch sogenannte anaplerotische Reaktionen wieder aufgefüllt werden. Bei Wachstum auf Zuckern erfolgt das Auffüllen des Oxalacetat-Pools durch die Carboxylierung von Pyruvat oder Phosphoenolpyruvat zu Oxalacetat. Die ATP-abhängige Bildung von Oxalacetat aus Pyruvat und Kohlenstoffdioxid bzw. HCO₃⁻ wird durch das Enzym Pyruvatcarboxylase (Pyc) katalysiert. Für die industrielle mikrobielle Produktion von Aminosäuren der Aspartat-Familie wie z.B. L-Lysin und der Glutamat-Familie wie z.B. L-Glutamat ist demnach eine hohe Aktivität der Pyc wichtig. Auch für die Produktion anderer Metabolite, die sich aus Intermediaten des Citratzyklus ableiten, ist eine hohe Pyc-Aktivität förderlich.

Die enzymatische Aktivität der nativen Pyc von C. *glutamicum* wird allosterisch unter anderem durch Aspartat gehemmt und hat daher bei hohen intrazellulären Aspartat-Konzentrationen nur eine begrenzte Aktivität. Damit ist auch die Produktion von L-Lysin und anderen, vom Oxalacetat abgeleiteten Produkten, begrenzt, da nur eine begrenzte Menge der Vorläufer-Metabolite bereitgestellt wird. Die bisher beschriebenen Pyc-Varianten führen nur zu einer moderaten Erhöhung der L-Lysinproduktion.

Die Veröffentlichung von Peters-Wendisch et al. "Pyruvate carboxylase is a major bottleneck for glutamate and lysine production by Corynebacterium glutamicum" im Journal of Molecular Microbiology and Biotechnology (2001) 32: 295-300 offenbart, dass die Deletion des *pyc-*Gens im Stamm C. *glutamicum* DG52-5 zu einer 60%igen Reduktion des Lysin-Titers führt, während die plasmid-vermittelte Überexpression des pyc-Gens im Stamm DG52-5 zu einem um 50% gesteigerten Lysin-Titer führt. Weiterhin wird gezeigt, dass die Deletion des pyc-Gens im Wildtyp-Stamm ATCC13032 die durch Tween 60 induzierte L-Glutamat-Produktion um etwa 50% reduziert, während die plasmid-basierte Überexpression des pyc-Gens die Glutamat-Bildung um 700% steigert. Die Wichtigkeit einer hohen Pyc-Aktivität für die Produktion von Lysin und Glutamat wurde später auch noch in anderen Publikationen gezeigt (Blombach et al. 2007 Applied Microbiology and Biotechnology 76: 615-623; Shirai et al. 2007 Microbial Cell Factories 6: 19; Neuner und Heinzle 2011 Biotechnology Journal 6: 318-329; Neuner et al. 2013 Journal of Biotechnology 163: 217-224; Guo et al. 2013 Biotechnology Letters 35: 943-950).

Auch für die Produktion anderer Metabolite außer Aminosäuren, die Intermediate des Citratzyklus darstellen oder sich von Intermediaten des Citratzyklus ableiten, ist eine hohe Pyc-Aktivität von großer Bedeutung. Die Veröffentlichungen von Shohei Okino et al. "An efficient succinic acid production process in a metabolically engineered Corynebacterium glutamicum strain" in Appl. Microbiol. Biotechnol. (2008) 81: 459-464 und Torben Hoefel et al. "Comparative reaction engineering studies for succinic acid production from sucrose by metabolically engineered Escherichia coli in fed-batch-operated stirred tank bioreactiors" in Biotechnol. J. 2012, 7, 1277-1287 offenbaren, dass die Expression bzw. Überexpression von Genen, kodierend für eine Pyruvatcarboxylase, zu einer Produktion oder gesteigerten Produktion von Succinat führt. Dies wird auch in weiteren Publikationen gezeigt (Li et al. 2016 Bioresource Technology 218: 217-223; Tajima et al. 2015 Applied and Environmental Microbiology 81: 929-937; Litsanov et al. 2012 Applied and Environmental Microbiology 78: 3325-3337; Meng et al. 2016 Microbial Cell Factories 15: 141). Für die Produktion von Malat mit *Thermobifida fusca* erwies sich eine erhöhte Pyc-Aktivität ebenfalls als förderlich (Deng et al. 2016 Biotechnology Progress 31: 14-20). Ebenfalls wurde für die Produktion von Diaminen, die sich aus Intermediaten des Citratzyklus ableiten, wie z.B. Putrescin (1,4-Diaminobutan) oder Cadaverin (1,5-Diaminopentan) eine Verstärkung der Pyc-Aktvität eingesetzt (Nguyen et al. 2015 Metabolites 5: 211-231; Kind et al. 2010 Metabolic Engineering 12: 341-351).

Ohnishi et al. (Applied Microbiology and Biotechnology (2002) 58: 217-223) haben die chromosomale Einführung des Aminosäureaustausches Prolin zu Serin an Position 458 der Pyc von *C*. *glutamicum* in den *C. glutamicum*-Stamm AHD2 beschrieben, der auf dem Wildtyp ATCC 13032 basiert und zwei Punktmutationen trägt, Val59Ala im Gen für die Homoserin-Dehydrogenase (hom) und Thr311Ile im Gen für die Aspartatkinase (*lysC*). Der Stamm AHP-3 mit der pyc-P458S-Mutation bildete 6% mehr L-Lysin als der Parentalstamm AHD-2. Die Autoren beschreiben, dass es für die Identifizierung der pyc-Mutation keinen bekannten selektierbaren Phänotyp gibt und sie vermutlich nur durch vergleichende Genomanalyse zu finden sei. Darüber hinaus wurde die Pyc-Variante Pyc-P458S noch nicht weiter charakterisiert.

Die Schrift US 7,300,777 B2, beschreibt einen Organismus, in welchem eine Pyruvatcarboxylase aus dem C. *glutamicum*-Stamm NRRL-B11474 mit mehreren Mutationen (M1V, E153D, A182S, A206S, H227R, A452G, D1120E) gegenüber der Pyc aus dem C. *glutami*cum-Stamm ATCC 21523 isoliert wurde. Mindestens eine der genannten Mutationen führt zu einer Pyc-Variante, die durch Aspartat in niedrigen Konzentrationen (1-10 mM) bis zu 2,5-fach in ihrer Aktivität stimuliert wird und bei höheren Aspartat-Konzentrationen wieder inhibiert wird bis zu maximal 30% der Aktivität in Abwesenheit von Aspartat. Bei 30 mM Aspartat zeigte die Pyc aus Stamm NRRL-B11474 noch die gleiche Aktivität wie in Abwesenheit von Aspartat, während die Pyc des Stammes ATCC 21523 bei 30 mM Aspartat nur noch 30% der Aktivität besaß, die sie in Abwesenheit von Aspartat zeigte. Die Auswirkung der feedback-resistenten Pyc-Variante aus C. *glutamicum* NRRL-B11474 auf die fermentative Produktion von Aminosäuren, insbesondere L-Lysin und L-Glutamat, wurde in der Schrift US 7,300,777 B2 jedoch nicht offenbart.

Die Deutsch Patentanmeldung 102012016716.4 offenbart ein Screeningverfahren, mit dem verbesserte Enzyme aufgefunden werden können.

Es ist daher die Aufgabe der Erfindung, einen Mikroorganismus, eine Pyruvatcarboxylase, ein für die Pyruvatcarboxylase kodierendes Gen, ein Plasmid oder Chromosom enthaltend dieses Gen sowie ein Verfahren zur Herstellung von Produkten, deren Biosynthese Oxalacetat als Vorstufe beinhaltet, zur Verfügung zu stellen, mit denen die Ausbeute, der Titer, die volumetrische Produktivität (g Produkt/Liter/Stunde) oder die spezifische Produktivität (g Produkt/Stunde/g Zelltrockenmasse) bei der Produktion von aus Oxalacetat abgeleiteten Produkten gesteigert werden kann. Weiterhin soll ein Screeningverfahren bereitgestellt werden, mit dem eine Pyruvatcarboxylase gefunden werden kann, welche die gestellte Aufgabe löst. Insbesondere soll die Produktion von Aminosäuren der Aspartat-Familie gesteigert werden, also L-Lysin, L-Aspartat, L-Asparagin, L-Threonin, L-Isoleucin und L-Methionin. Weiterhin soll die Produktion von Aminosäuren der Glutamat-Familie, wie L-Glutamat, L-Glutamin, L-Arginin oder L-Prolin, von Intermediaten wie Salzen und Säuren des Citrat-Zyklus beispielsweise Succinat, Malat, Fumarat, 2-Oxoglutarat, Citrat oder Isocitrat, von Diaminen wie zum Beispiel 1,5-Diaminopentan oder 1,4-Diaminobutan oder auch von weiteren Produkten wie Itaconat, Ectoin, gamma-Aminobutyrat, Butanol, 1-Propanol, L-Citrullin, L-Ornithin, D-Arginin oder 4-Hydroxyprolin gesteigert werden.

Ausgehend vom Oberbegriff des Anspruchs 1 und den nebengeordneten Ansprüchen wird die Aufgabe erfindungsgemäß gelöst mit den im kennzeichnenden Teil des Anspruchs 1 und der nebengeordneten Ansprüche angegebenen Merkmalen.

Mit dem Mikroorganismus, der Pyruvatcarboxylase, dem für die Pyruvatcarboxylase kodierenden Gen, dem Plasmid oder Chromosom enthaltend dieses Gen, sowie mit dem Herstellungsverfahren und dem Screeningverfahren kann die Ausbeute von Produkten, deren Biosynthese Oxalacetat als Vorstufe beinhalten, gesteigert werden. Insbesondere kann die Produktion von Aminosäuren der Oxalacetat/Aspartat-Familie gesteigert werden, also L-Lysin, L-Aspartat, L-Asparagin, L-Threonin, L-Isoleucin, L-Methionin. Weiterhin kann die Produktion von Aminosäuren der Glutamat-Familie wie L-Glutamat, L-Glutamin, L-Arginin oder L-Prolin, von Intermediaten wie Salzen und Säuren des Citrat-Zyklus, beispielsweise Succinat, Malat, Fumarat oder 2-Oxoglutarat, Citrat oder Isocitrat, von Diaminen beispielsweise. 1,5-Diaminopentan oder 1,4-Diaminobutan oder auch von weiteren Produkten wie Itaconat, Ectoin, gamma-Aminobutyrat, Butanol, 1-Propanol, L-Citrullin, L-Ornithin, D-Arginin oder 4-Hydroxyprolin gesteigert werden. Vorteilhafte Weiterbildungen der Erfindung sind in den Unteransprüchen angegeben.

Im Folgenden wird die Erfindung in ihrer allgemeinen Form beschrieben, ohne dass dies einschränkend auszulegen ist.

Überraschenderweise wurde gefunden, dass eine gegenüber dem Stamm *C. glutamicum* ATCC 13032 *lysC*^{T311I} veränderte Pyruvatcarboxylase mit einem Austausch von Threonin in Position 132 durch Alanin, und ein für diese Pyruvatcarboxylase kodierendes, genetisch verändertes Gen, ein Plasmid, enthaltend dieses Gen, sowie ein genetisch veränderter Mikroorganismus enthaltend dieses Gen oder Plasmid sowie das Herstellungsverfahren und das Screeningverfahren die gestellten Aufgaben löst. Beispielsweise wird für die Produktion von L-Lysin gegenüber dem Stamm ATCC 13032 *lys*C^{T311I} eine um 7 bis 19% gesteigerte Endkonzentration an L-Lysin festgestellt.

Erfindungsgemäß wird ein Gen einer Identität von mindestens 85% des Gens nach Sequenz Nr.1, kodierend für eine Pyruvatcarboxylase zur Verfügung gestellt, welches ausgehend von dem Gen einer mindestens 85%igen Identität von Sequenz Nr.1 in Position 394-396 einen Austausch des für Threonin-132 kodierenden Tripletts gegen ein Triplett kodierend für Alanin besitzt. Position 394-396 kodiert daher für Alanin. Von der erfindungsgemäßen DNA sind Sequenzen einer 85%igen bis 100%igen Identität umfasst. Vorzugsweise ist die Identität 85% bis 90%. Besonders bevorzugt 91% 92%, 93%, 94%, 95%, 96%, 97%, 98% oder 99%. Ganz besonders bevorzugt ist das Gen nach Sequenz Nr. 1.

Identität wie hierin verwendet, kann durch die Gleichung I (%) = [1-V/X] x 100, definiert werden, worin I Identität bedeutet, X die Gesamtzahl an Nukleobasen der Vergleichssequenz ist und V die Anzahl an unterschiedlichen Nukleobasen der zu betrachtenden Sequenz bezogen auf die Vergleichssequenz ist. Auf jeden Fall sind mit dem Begriff Nukleinsäuresequenzen, welche für Polypeptide kodieren, alle Sequenzen umfasst, die nach Maßgabe der Degeneration des genetischen Codes möglich erscheinen.

Weiterhin wird erfindungsgemäß ein Vektor, vorzugsweise ein Plasmid, enthaltend dieses Gen zur Verfügung gestellt. Dabei kommt grundsätzlich jeder Leervektor oder jedes Leerplasmid als Ausgangsvektor oder Ausgangsplasmid in Betracht. Beispielsweise kann als Leervektor das Plasmid pAN6, wie es in der Veröffentlichung von Frunzke et al., Molecular Microbiology 2008, 67:305-322 beschrieben wird, eingesetzt werden, bei dem das erfindungsgemäße Gen inseriert ist.

Erfindungsgemäß wird auch ein genetisch verändertes Chromosom zur Verfügung gestellt, welches die erfindungsgemäße DNA enthält. Dabei soll die erfindungsgemäße DNA so in das Chromosom inseriert sein, dass die Funktion der für die Lebensfähigkeit des Mikroorganismus relevanten Gene nicht beeinträchtigt oder zerstört wird.

Durch Expression dieses Gens nach Sequenz Nr. 1 wird eine erfindungsgemäße Pyruvatcarboxylase mit einer mindestens 90%igen Sequenzidentität zu der Pyruvatcarboxylase nach Sequenz Nr. 2 erhalten, bei welcher ausgehend von der Pyruvatcarboxylase des Stammes ATCC 13032 *lys*C^{T311I} das Threonin an Position 132 durch Alanin ersetzt ist (*pyc*^{T132A}). In Position der Pyruvatcarboxylase 132 befindet sich daher erfindungsgemäß Alanin. Von der erfindungsgemäßen Pyruvatcarboxylase sind Pyruvatcarboxylasen einer 90%igen bis 100%igen Identität zu der Sequenz Nr. 2 umfasst. Vorzugsweise ist die Identität 95%, 96% oder 97%, besonders bevorzugt 98% oder 99% der erfindungsgemäß veränderten Pyruvatcarboxylase von Sequenz Nr. 2. Ganz besonders bevorzugt ist die Pyruvatcarboxylase nach Sequenz Nr. 2.

Der Ausdruck Identität wie hierin verwendet, kann durch die Gleichung I (%) = [1-V/X] x 100, definiert werden, worin I Identität bedeutet, X die Gesamtzahl an Aminosäuren der Vergleichssequenz ist und V die Anzahl an unterschiedliche Aminosäuren der zu betrachtenden Sequenz bezogen auf die Vergleichssequenz ist. Folgende Sequenzen sind im Sequenzprotokoll gelistet:
Seq. Nr.1: Erfindungsgemäß veränderte DNA-Sequenz der Plasmid-basierten Variante, codierend für die erfindungsgemäß veränderte Pyruvatcarboxylase.
Seq. Nr. 2: Aminosäuresequenz der erfindungsgemäß veränderten Pyruvatcarboxylase
Seq. Nr.3: DNA-Sequenz des Referenzstammes ATCC 13032 *lysC*^{T311I} für die Wildform der Pyruvatcarboxylase.
Seq. Nr.4: Aminosäuresequenz der Pyruvatcarboxylase des Referenzstammes ATCC 13032 *lys*C^{T311I} für die Wildform der Pyruvatcarboxylase
Seq.Nr.5: DNA-Sequenz der erfindungsgemäßen chromosomalen Variante DNA-pyc-A394G-G402C.

In einer vorteilhaften Weiterbildung der Erfindung kann die Expression der erfindungsgemäßen Gene verstärkt werden. Dazu können beispielhaft aber nicht beschränkend stärkere Promotoren verwendet werden, die Anzahl der Genkopien kann erhöht werden oder es kann die Ribosomenbindestelle verändert werden, um die Translation der Boten-RNA zu steigern. Die für die Durchführung dieser Methoden zu verwendenden Verfahren sind dem Fachmann bekannt.

Weiterhin ist ein Mikroorganismus Gegenstand der Erfindung, welcher ein erfindungsgemäßes Gen oder einen erfindungsgemäßen Vektor enthält. Dieser genetisch veränderte Mikroorganismus ist vorzugsweise ein coryneformes Bakterium. Als coryneforme Bakterien können beispielsweise *Corynebacterium glutamicum, Corynebacterium acetoglutamicum, Corynebacterium acetoacidophilum, Corynebacterium melassecola, Corynbacterium thermoaminogenes, Corynebacterium efficiens, Brevibacterium flavum oder Brevibacterium lactofermentum* genannt werden.

Erfindungsgemäß besonders bevorzugte Zellen sind diejenigen der Gattungen *Corynebacterium, Brevibacterium, Escherichia, Bacillus, Lactobacillus, Lactococcus, Zymomonas, Methylobacterium, Ralstonia, Clostridium, Candida, Pichia, Kluyveromyces, Saccharomyces* und *Yarrowia,* wobei *Corynebacterium glutamicum, Corynebacterium efficiens, Brevibacterium flavum, Brevibacterium lactofermentum, Escherichia coli, Saccharomyces cerevisiae, Kluyveromyces lactis, Candida blankii, Candida rugosa, Zymomonas mobilis, Yarrowia lipolytica, Methylobacterium extorquens, Ralstonia eutropha* und *Pichia pastoris* besonders bevorzugt sind. Erfindungsgemäß am meisten bevorzugte Zellen sind solche der Gattung *Corynebacterium* und *Escherichia,* wobei *Corynebacterium glutamicum* und *Escherichia coli* ganz besonders bevorzugte Bakterienstämme sind.

Insbesondere in dem Fall, in dem das Produkt L-Lysin ist, können sich die gentechnisch veränderten Zellen insbesondere von Zellen ausgewählt aus der Gruppe bestehend aus *Corynebacterium glutamicum* ATCC 13032, *Corynebacterium acetoglutamicum* ATCC 15806, *Corynebacterium acetoacidophilum* ATCC13870, *Corynebacterium melassecola* ATCC17965, *Corynebacterium thermoaminogenes* FERM BP-1539, *Brevibacterium flavum* ATCC14067, *Brevibacterium lactofermentum* ATCC13869 und *Brevibacterium divaricatum* ATCC14020, und daraus hergestellte L-Aminosäuren produzierende Mutanten bzw. Stämme wie beispielsweise die L-Lysin produzierenden Stämme *Corynebacterium glutamicum* FERM-P 1709, *Brevibacterium flavum* FERM-P 1708, *Brevibacterium lactofermentum* FERM-P 1712, *Corynebacterium glutamicum* FERM-P 6463, *Corynebacterium glutamicum* FERM-P 6464 und *Corynebacterium glutamicum* DSM 5715 oder wie beispielsweise der L-Methionin produzierende Stamm *Corynebacterium glutamicum* ATCC21608 ableiten. Als Beispiele geeigneter *Escherichia* coli-Stämme seien *Escherichia coli* AJ11442 (siehe JP 56-18596 und US 4,346,170), *Escherichia coli*-Stamm VL611 und *Escherichia coli*-Stamm WC196 (siehe WO-A-96/17930) genannt.

Erfindungsgemäß wird ein Verfahren zur Herstellung von Produkten, deren Biosynthese Oxalacetat als Vorstufe beinhaltet, zur Verfügung gestellt.

Dazu wird ein genetisch veränderter Mikroorganismus, enthaltend ein für die Pyruvatcarboxylase codierendes Gen mit der genetischen Veränderung, kodierend für den Austausch T132A im Protein, für die Produktion von aus Oxalacetat abgeleiteten Stoffwechselprodukten verwendet.

Hierzu wird ein Gen mit einer mindestens 85%igen Identität zu der Sequenz Nr.1, bei der in den Positionen 394-396 das für Threonin kodierende Triplett gegen ein Triplett kodierend für Alanin ausgetauscht ist, in den Mikroorganismus eingeführt.

Von dem erfindungsgemäßen Verfahren ist die Verwendung eines Gens mit einer mindestens 85%igen bis 100%igen Identität zu dem Gen nach Sequenz Nr.1 umfasst.

Vorzugsweise wird ein genetisch veränderter Mikroorganismus mit einem Gen einer Identität von mindestens 85% bis 90% zu Sequenz Nr. 1 verwendet. Besonders bevorzugt wird ein Gen mit einer Identität von 91%, 92%, 93%, 94%, 95%, 96%, 97%, 98% oder 99% zu Sequenz Nr. 1 für das Herstellungsverfahren verwendet. Ganz besonders bevorzugt ist das Gen nach Sequenz Nr. 1.

Das erfindungsgemäß eingesetzte für die Pyruvatcarboxylase kodierende Gen kann dabei chromosomal oder in einem Vektor, vorzugsweise einem Plasmid, verwendet werden.

Das Gen wird exprimiert und die erfindungsgemäße Pyruvatcarboxylase mit einer Identität von mindestens 90% zu der Pyruvatcarboxylase nach Sequenz Nr. 2, bei der in Position 132 Threonin durch Alanin ersetzt ist, bewirkt die erhöhte Produktion von Oxalacetat abgeleiteten Stoffwechselprodukten.

Von dem erfindungsgemäßen Verfahren ist die Verwendung einer Pyruvatcarboxylase mit einer 90%igen bis 100%igen Identität zu der Pyruvatcarboxylase nach Sequenz Nr. 2 umfasst.

Vorzugsweise ist die Identität der erfindungsgemäß verwendeten Pyruvatcarboxylase 95%, 96% oder 97%, besonders bevorzugt 98% oder 99% zu Sequenz Nr. 2. Ganz besonders bevorzugt ist die Verwendung der Pyruvatcarboxylase nach Sequenz Nr. 2. In einer bevorzugten Ausführungsform wird das erfindungsgemäße Gen verstärkt exprimiert.

Die so erhaltenen genetisch veränderten Mikroorganismen werden fermentiert.

Als Produktionsorganismus kann vorzugsweise ein Organismus aus der Gruppe bestehend aus den Gattungen *Corynebacterium, Brevibacterium, Escherichia, Bacillus, Lactobacillus, Lactococcus, Zymomonas, Methylobacterium, Ralstonia, Clostridium, Candida, Pichia, Kluyveromyces, Saccharomyces* und *Yarrowia,* wobei *Corynebacterium glutamicum, Corynebacterium efficiens, Brevibacterium flavum, Brevibacterium lactofermentum, Escherichia coli, Saccharomyces cerevisiae, Kluyveromyces lactis, Candida blankii, Candida rugosa, Zymomonas mobilis, Yarrowia lipolytica, Methylobacterium extorquens, Ralstonia eutropha* und *Pichia pastoris* besonders bevorzugt sind. Erfindungsgemäß am meisten bevorzugte Zellen sind solche der Gattung *Corynebacterium* und *Escherichia,* wobei *Corynebacterium glutamicum* und *Escherichia coli ganz* besonders bevorzugte Bakterienstämme sind.

Insbesondere in dem Fall, in dem der Metabolit L-Lysin ist, können die gentechnisch veränderten Zellen bzw. Mikroorganismen insbesondere von Zellen ausgewählt aus der Gruppe bestehend aus *Corynebacterium glutamicum* ATCC13032, *Corynebacterium acetoglutamicum* ATCC15806, *Corynebacterium acetoacidophilum* ATCC13870, *Corynebacterium melassecola* ATCC17965, *Corynebacterium thermoaminogenes* FERM BP-1539, Brevibacterium *flavum* ATCC14067, *Brevibacterium lactofermentum* ATCC13869 und *Brevibacterium divaricatum* ATCC14020, und daraus hergestellte L-Aminosäuren produzierende Mutanten bzw. Stämme wie beispielsweise die L-Lysin produzierenden Stämme *Corynebacterium glutamicum* FERM-P 1709, *Brevibacterium flavum* FERM-P 1708, *Brevibacterium lactofermentum* FERM-P 1712, *Corynebacterium glutamicum* FERM-P 6463, *Corynebacterium glutamicum* FERM-P 6464 und *Corynebacterium glutamicum* DSM 5715 oder wie beispielsweise der L-Methionin produzierende Stamm *Corynebacterium glutamicum* ATCC21608 ableiten. Als Beispiele geeigneter *Escherichia* coli-Stämme seien *Escherichia coli* AJ11442 (siehe JP 56-18596 und US 4,346,170), *Escherichia coli*-Stamm VL611 und *Escherichia coli-*Stamm WC196 (siehe WO-A-96/17930) eingesetzt werden.

Mit dem erfindungsgemäßen Verfahren können insbesondere Aminosäuren der Aspartat-Familie gesteigert werden, also L-Lysin, L-Aspartat, L-Asparagin, L-Threonin, L-Isoleucin und L-Methionin hergestellt werden. Weiterhin kann die Produktion von Aminosäuren der Glutamat-Familie, wie L-Glutamat, L-Glutamin, L-Arginin oder L-Prolin, von Intermediaten des Citrat-Zyklus, wie z.B. Succinat, Fumarat, Malat, Citrat, Isocitrat oder 2-Oxoglutarat, von Diaminen z.B. Diaminopentan oder Diaminobutan oder auch anderen Produkten wie Itaconat, Ectoin, gamma-Aminobutyrat, Butanol, 1-Propanol, L-Citrullin, L-Ornithin, D-Arginin oder 4-Hydroxyprolin gesteigert werden.

Das durch Fermentation hergestellte und in den Kulturüberstand sekretierte Produkt wird dann angereichert und isoliert.

Weiterhin wird erfindungsgemäß ein Screeningverfahren zur Verfügung gestellt, mit welchem eine erfindungsgemäße Pyruvatcarboxylase aufgefunden werden kann.

Dazu wird erfindungsgemäß ein Vektor hergestellt, welcher ein Gen kodierend für eine Pyruvatcarboxylase enthält.

Zu diesem Zweck werden Gene, die für eine Pyruvatcarboxylase kodieren, in vitro mutagenisiert und diese Genbibliothek in einem weiteren Schritt in einen Vektor inseriert.

Die resultierenden pyc-tragenden Vektoren werden in einem weiteren Schritt in Zellen eines Mikroorganismus eingebracht, der einen Metabolitsensor enthält, der bei erhöhter Metabolitkonzentration die Synthese eines Fluoreszenzproteins bewirkt. Dieser Metabolitsensor muss ein Produkt detektieren, dessen Biosynthese Oxalacetat als Vorstufe beinhaltet.

Nach Wachstum der Zellbibliothek wird eine Selektion der Einzelzellen durchgeführt, die eine erhöhte Synthese eines Fluoreszenzproteins aufweisen und die daher eine erhöhte spezifische Fluoreszenz (= Fluoreszenz pro Zelle) aufweisen.

Die Vektoren, die ein für eine Pyruvatcarboxylase kodierendes Gen enthalten, werden aus Mikroorganismen mit erhöhter oder der höchsten Fluoreszenz isoliert und die pyc-Gene werden einer DNA-Sequenzanalyse unterzogen, um die Unterschiede zur nativen pyc-Sequenz zu identifizieren sowie die sich daraus ableitenden Änderungen in der Aminosäuresequenz der Pyruvatcarboxylase.

Die isolierten Vektoren werden erneut in den Mikroorganismus mit dem Metabolitsensor eingebracht und überprüft, ob die Expression des mutierten pyc-Gens wiederum zu einer erhöhten Fluoreszenz führt. Nur solche Vektoren, die wiederum zu einer erhöhten Fluoreszenz führen werden weiterverfolgt.

Mit diesen Verfahrensschritten kann ermittelt werden, welche pyc-Variante die höchste oder eine erhöhte Produktion des gewünschten Produkts hervorbringt.

Die nach diesem Verfahren isolierten Vektoren enthalten Gene, die für Pyruvatcarboxylasen kodieren.

Diese in den Vektoren enthaltenen Gene, die eine Pyruvatcarboxylase kodieren, können nach dem Fachmann bekannten Verfahren zur Produktion von Aminosäuren, Nukleotiden oder Intermediaten benutzt werden.

Hierfür können beispielhaft die nachfolgenden, dem Fachmann bekannten Verfahrensschritte durchgeführt werden.

Die Gene, die für Pyruvatcarboxylasen kodieren, können auf dem Vektor in einen geeigneten Produktionsstamm eingeführt werden, der dann zur besseren Produktion des gewünschten Produktes befähigt ist.

Alternativ können die Gene, die für Pyruvatcarboxylasen kodieren, in Vektoren eingebaut werden, die bereits zur Produktion des gewünschten Produktes dienen und zu einer zusätzlichen Steigerung der Produktbildung führen.

In einer weiteren Ausführungsform können die Gene, die für Pyruvatcarboxylasen kodieren, in das Chromosom des Produktionsstammes eingeführt werden, der dann zur besseren Produktion des gewünschten Produktes befähigt ist.

Im Folgenden werden einzelne Schritte des Verfahrens in ihren möglichen Ausgestaltungen genauer ausgeführt, ohne dass dies beschränkend auszulegen ist:

### Erzeugung von in vitro mutagenisierten Genen für eine Pyruvatcarboxylase.

Bei dem Ausgangsorganismus, aus dem das zu verändernde Gen bezogen wird, kann es sich um jeden beliebigen Mikroorganismus handeln, der ein Gen für eine Pyruvatcarboxylase kodiert, beispielsweise um ein Bakterium, wie ein Corynebakterium, oder eine Hefe, wie beispielsweise Saccharomyces cerevisiae. Insbesondere kann Corynebakterium glutamicum als Ausgangsorganismus eingesetzt werden.

Die Mutagenisierung kann vorzugsweise durch ungerichtete Methoden durchgeführt werden, wie beispielsweise durch eine fehlerhafte Polymerasekettenreaktion, die nach dem Stand der Technik bekannt sind.

Zur Einführung der ortsunspezifischen Mutationen in die plasmidkodierten Gene der Pyruvatcarboxylasen wird bevorzugt eine in vitro Mutagenese unter Zuhilfenahme einer fehlerhaften Polymerasekettenreaktion (PCR) durchgeführt. Dabei wird das zu mutierende Gen einer PCR unter Verwendung einer Polymerase unterzogen, die abhängig von den Bedingungen der Reaktion einzelne Nucleotide gegenüber der Wildform falsch in die synthetisierten Gene einbaut (Tindall, K.R. and T.A. Kunkel, Fidelity of DNA synthesis by the Thermus aquaticus DNA polymerase. Biochemistry, 1988. 27(16): p. 6008-13). Eine häufige Variante dieser Methode beinhaltet die Verwendung von Mangan(II)-Ionen oder von Nukleotidanaloga im PCR-Ansatz (Cadwell R. C et al. (1992) PCR Methods Appl. 2:28-33./Leung D. W. et al. (1989) Techniques 1:11-15). Diese Techniken zur Mutationseinführung werden als "Error-Prone-PCR (epPCR)" bezeichnet (Labrou NE. Random mutagenesis methods for in vitro directed enzyme evolution. Curr Protein Pept Sci. 2010;11:91-100). Die Mutationen können beispielsweise Punktmutationen sein, es können z.B. Substitutionen, Deletionen oder Insertionen durch die Polymerase erzeugt werden. Die Mutationsrate beträgt zwischen 1-40 Mutationen pro 1 kb, bevorzugt 1-5 Mutationen pro 1 kb.

### Klonierung der mutierten Gene für eine Pyruvatcarboxylase in einen Vektor:

Die mutierten Gene werden in einen Vektor inseriert. Als Vektoren können alle bekannten Plasmidkörper, Transposons, Insertionselemente oder Phagen als Rohmaterial eingesetzt werden, in die die mutagenisierten Gene inseriert wurden. Beispielhaft aber nicht beschränkend, können Vektoren, die sich von pBR322, pACYC184 (Bartolome et al.; Gene 102, 75-78 (1991)), oder pTrc99A (Amann et al.: Gene 69: 301-315 (1988)) oder pSC101 (Vocke and Bastia, Proceedings of the National Academy of Sciences USA 80 (21): 6557-6561 (1983)) ableiten, verwendet werden. Derartige genetische Systeme sind beispielsweise in den Patentschriften US 4,822,738, US 5,804,414 und US 5,804,414 beschrieben. In gleicher Weise können auch Vektoren wie pZ1, pXZ10142, pEKEx2, pEKEx3, oder pEC-t19mob2 (zitiert im "Handbook of Corynebacterium glutamicum" (Herausgeber: L. Eggeling und M. Bott)) verwendet werden, die für das erfindungsgemäße Verfahren eingesetzt werden.

Transfer der Plasmid-basierten pyc-"Bibliothetek" in einen Organismus, der einen geeigneten Metabolitsensor trägt, in dem die Synthese eine Fluoreszenzproteins in Abhängigkeit von der Metabolitkonzentration gesteuert wird. Der Metabolitsensor detektiert die Konzentration eines Metaboliten, dessen Biosynthese Oxalacetat als Vorstufe beinhaltet.

Der zur Transformation benutzte Mikroorganismus kann jeder beliebige Mikroorganismus sein, in dem ein Metabolitsensor mit den oben beschriebenen Merkmalen funktional ist. Beispielhaft können Bakterien oder Hefen, beispielsweise E. coli, Corynebakterium glutamicum oder Saccharomyces cerevisiae genannt werden. Als Metabolitsensoren können jegliche Sensoren eingesetzt werden, die einen Metaboliten detektieren, dessen Biosynthese Oxalacetat als Vorstufe beinhaltet, beispielsweise Biosensoren für L-Lysin, L-Aspartat, L-Asparagin, L-Threonin, L-Isoleucin, L-Methionin, L-Glutamat, L-Glutamin, L-Arginin, L-Prolin, Succinat, Malat, Fumarat, 2-Oxoglutarat, Citrat, Isocitrat, 1,5-Diaminopentan, 1,4-Diaminobutan, Itaconat, Ectoin, gamma-Aminobutyrat, Butanol, 1-Propanol, L-Citrullin, L-Ornithin, D-Arginin oder 4-Hydroxyprolin. Beispielsweise können bekannte Metabolitsensoren, wie pSenLys, pSenArg, oder pJC1-lrp-brnF-eyfp eingesetzt werden. Der Metabolitsensor umfasst eine für ein Autofluoreszenzprotein kodierende Gensequenz, wobei die Expression des Autofluoreszenzproteins von der intrazellulären Konzentration eines bestimmten Metaboliten abhängig ist. Dabei erfolgt die Kontrolle der Expression der für das Autofluoreszenzprotein kodierenden Gensequenz in Abhängigkeit von der intrazellulären Konzentration des bestimmten Metaboliten auf der Ebene der Transkription. In Abhängigkeit von der intrazellulären Konzentration des jeweiligen Metaboliten wird mithin mehr oder weniger mRNA gebildet, die von den Ribosomen unter Bildung des Autofluoreszenzproteins translatiert wird.

Als Mikroorganismus können alle in der Biotechnologie eingesetzten Stämme verwendet werden, in die die Plasmide, die die mutagenisierten Gene, die für eine Pyruvatcarboxylase, kodieren eingeführt wurden. Beispielhaft können coryneforme Bakterien, Enterobakterien oder Hefen, wie beispielsweise E.coli, Corynebakterium glutamicum, oder Saccharomyces cerevisiae genannt werden.

Die Vektoren, vorzugsweise Plasmide, die die erfindungsgemäß erhaltenen Mutationen in Genen der Pyruvatcarboxylasen enthalten, werden durch Transformation in einen Mikroorganismus, wie beispielsweise E. coli, C. glutamicum oder Saccharomyces cerevisiae eingebracht. Der Begriff Transformation umfasst sämtliche Methoden zur Übertragung von Polynukleotiden, insbesondere DNA, in einen gewünschten Mikroorganismus. Hierzu gehören unter anderem die Verwendung von isolierter DNA bei der Transformation, Elektrotransformation bzw. Elektroporation, die Übertragung durch Zellkontakt wie bei der Konjugation oder die Übertragung von DNA mittels Partikelbeschuss.

Wachstum der Zellen und Selektion der Einzelzellen mit den Pyc-Varianten, die zu einer hohen oder der höchsten Fluoreszenz führen.

Die Mikroorganismen mit dem Metabolitsensor und den mutierten Pyc-Varianten werden in einem geeigneten Kulturmedium kultiviert, so dass Wachstum stattfindet. Im nachfolgenden Verfahrensschritt des erfindungsgemäßen Verfahrens werden einzelne transformierte Zellen in der Zellsuspension mit erhöhter intrazellulärer Konzentration von Metaboliten durch Nachweis einer erhöhten spezifischen Fluoreszenz (= Fluoreszenz pro Zelle) identifiziert, und zwar mit Hilfe der Durchflusszytometrie. Dazu wird die Zellsuspension elektromagnetischer Strahlung derjenigen Frequenz ausgesetzt, welche das Autofluoreszenzprotein des Metabolitsensors zur Emission von Licht anregt. Als Autofluoreszenzprotein bevorzugt sind Proteine, die aus Gensequenzen abgelesen werden, welche für fluoreszierende Proteine beispielsweise der Gattung Aequora, wie das Green Fluorescent Protein (GFP), und Varianten davon, die in einem anderen Wellenlängenbereich fluoreszieren (z.B. Yellow Fluorescent Protein, YFP; Blue Fluorescent Protein, BFP; Cyan Fluorescent Protein, CFP) oder deren Fluoreszenz verstärkt ist (enhanced GFP oder EGFP, beziehungsweise EYFP, EBFP oder ECFP), kodieren. Ferner können erfindungsgemäß auch Gensequenzen verwendet werden, welche für andere autofluoreszierende Proteine, z.B. DsRed, HcRed, AsRed, AmCyan, ZsGreen, AcGFP, ZsYellow, wie sie von BD Biosciences, Franclin Lakes, USA, bekannt sind, kodieren. Ferner können auch Gensequenzen für Protein verwendet werden, deren Autofluoreszenz auf Flavin-Cofaktoren oder anderen Cofaktoren mit Autofluroeszenz basiert. Das besonders bevorzugte Autofluoreszenzprotein ist dabei EYFP bzw. das dafür kodierende Gen.

Gemäß einer besonderen Ausgestaltung des erfindungsgemäßen Verfahrens erfolgt im Anschluss, vorzugsweise im unmittelbaren Anschluss an das Identifizieren der Zellen mit erhöhter Fluoreszenz ein weiterer Verfahrensschritt, in dem die identifizierten Zellen aus der Zellsuspension abgetrennt werden, wobei dieses Abtrennen vorzugsweise mittels Fluoreszenz-aktivierter Zellsortierung (FACS, fluorescence-activated cell sorting) erfolgt. Einzelheiten zur Analyse von Zellsuspensionen mittels Durchflusszytometrie können beispielsweise Sack U, Tarnok A, Rothe G (Hrsg): Zelluläre Diagnostik, Grundlagen, Methoden und klinische Anwendungen der Durchflusszytometrie, Basel, Karger, 2007, Seiten 27 - 70 entnommen werden.

Mittels des erfindungsgemäßen Verfahrens ist es daher möglich, aus einer Suspension von Zellen, die eine Genbank mutierter plasmidkodierter Pyruvatcarboxylasen enthalten, gezielt diejenigen Zellen zu isolieren, in denen die Pyruvatcarboxylase zu einer gesteigerten intrazellularen Konzentration des Metaboliten führen, der durch den in dieser Zellen vorhandenen Metabolitsensor detektiert wird. Die erhöhte intrazelluläre Konzentration des Metaboliten führt in der Folge zu einer verstärkten Expression des Gens für das Autofluoreszenzprotein und damit zu einer erhöhten spezifischen Fluoreszenz.

Die Einzelzellen mit erhöhter Fluoreszenz, welche durch fluoreszenz-aktivierter Zellsortierung isoliert wurden, werden in einem geeigneten Kultumedium vermehrt. Anschließend wird aus den Klonen die Plasmid-DNA isoliert und das im Plasmid enthaltenen pyc-Gens einer DNA-Sequenzanalvse unterzogen, um die Mutation(en) zu identifizieren, welche diese pyc-Variante trägt. Beispielhaft wird erfindungsgemäß die Variante pyc-T132A identifiziert. Die mit dem erfindungsgemäßen Verfahren erhaltenen Nukleinsäuren und Pyruvatcarboxylasen können für die verbesserte Herstellung von Produkten verwendet werden, deren Biosynthese Oxalacetat als Vorstufe beinhaltet.

### Transfer der mutierten pvc-Gene in einen geeigneten Vektor oder chromosomal integriert in einen geeigneten Produktionsstamm:

Die identifizierten pyc-Varianten können entweder plasmid-basiert oder chromosomal inseriert in Mikroorganismen-Stämme eingebracht werden, die bereits vorher für die Produktion eines Metaboliten optimiert wurden, dessen Biosynthese Oxalacetat als Vorstufe beinhaltet.

Als Vektoren kann jeder beliebige im gewählten Wirtsorganismus replizierbare Vektor verwendet werden, vorzugsweise Plasmide. Für die chromosomale Integration können bekannte Verfahren mittels homologer Rekombination, mittels einer Kombination aus Recombineering und FACS (Binder et al. 2013 Nucleic Acids Research Vol. 41, No. 12, 6360-6369), oder mittels CRISPR/Cas verwendet werden.

Die Stämme, die die verbesserten Pyruvatcarboxylasen exprimieren, werden unter den für die Produktion des aus Oxalacetat abgeleiteten Zielprodukts geeigneten Kultivierungsbedingungen fermentiert und anschließend das Zielprodukt mit geeigneten Verfahren isoliert oder alternativ die gesamte Kulturbrühe mit den Zellen zu einem vermarktungsfähigen Produkt konvertiert.

Im Folgenden werden experimentelle Ergebnisse anhand von Figuren dargestellt, die die Erfindung exemplarisch wiedergeben.

Es zeigt:
- Fig.1: Wachstum von *C. glutamicum* ATCC 13032 *lysC*^{T311I} mit chromosomal codierter Pyc^{T132A}.
- Fig.2:: L-Lysin-Produktion des Stammes C. *glutamicum* ATCC 13032 *lys*C^{T311I} mit chromosomal codierter Pyc^{T132A}.
- Fig.3:: Wachstum von C. *glutamicum* ATCC 13032 *lys*C^{T311I} Δ*pyc* mit dem Plasmid pAN6-*pyc*^{T132A}.
- Fig.4:: L-Lysin-Produktion des Stammes C. *glutamicum* ATCC 13032 *lys*C^{T311I} Δ*pyc* mit dem Plasmid pAN6-*pyc*^{T132A}.

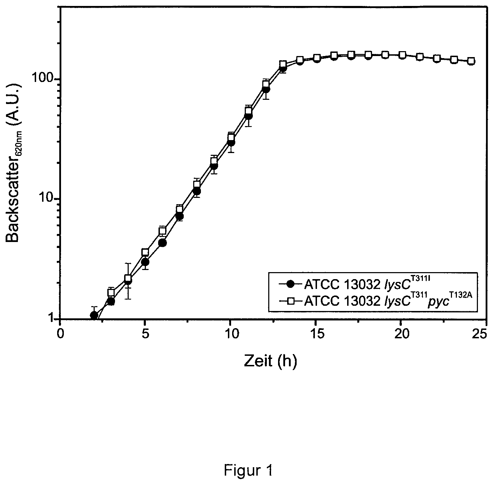

In Figur 1 wird das Wachstum des Stammes C. *glutamicum* ATCC 13032 *lys*C^{T311I} mit der chromosomal codierten Pyc-Variante Pyc^{T132A} dargestellt. In Figur 1 zeigt die Abszisse die Zeit in Stunden (h) und die Ordinate den Wert für Backscatter bei 620 nm (A.U.) als Maß der Zelldichte. Als Kontrolle diente der Stamm C. *glutamicum* ATCC 13032 *lys*C^{T311I} mit nativer Pyc, also mit Threonin an Position 132. Alle Stämme wurden in CGXII Minimalmedium mit 4% (wt/vol) Glucose in einem BioLector^{®} System bei 30 °C und 1200 rpm für 24 h kultiviert.

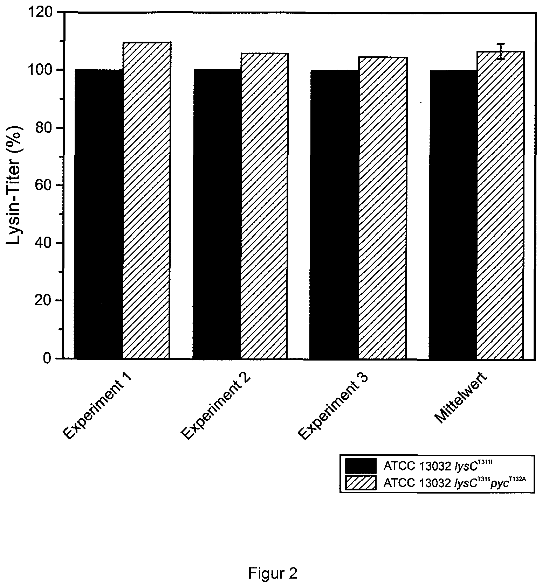

Figur 2 zeigt die L-Lysin-Produktion des Stammes C. *glutamicum* ATCC 13032 *lys*C^{T311I} mit der chromosomal codierten Pyc-Variante Pyc^{T132A}. Darin bezeichnet die Abszisse drei unabhängige Replikate sowie den Mittelwert aus den drei Experimenten und die Ordinate die prozentuale Lysin-Konzentration, wobei die Lysin-Konzentration des Kontrollstammes ATCC 13032 *lys*C^{T311I} (schwarze Balken) in den drei unabhängigen Replikaten jeweils als 100% gesetzt wurde. Die L-Lysin Konzentrationen für den Stamm ATCC 13032 *lys*C^{T311I} *pyc*^{T132A} sind als schraffierte Balken dargestellt. Nach Kultivierung der Zellen in einem BioLector^{®} System für 24 h (s. Fig.1) wurden die Zellen geerntet und die L-Lysin Konzentration im Überstand der jeweiligen Kulturen via reversed-phase HPLC mit ortho-Phthaldialdehyd-Derivatisierung bestimmt. In jedem der drei Replikate zeigt der Stamm mit der mutierten Pyc-Variante Pyc-T132A einen höheren Lysin-Titer als der Vergleichsstamm mit nativer Pyc. Es ist eine Steigerung der finalen L-Lysin-Konzentration um durchschnittlich 7% gegenüber C. *glutamicum* ATCC 13032 *lys*C^{T311I} zu beobachten.

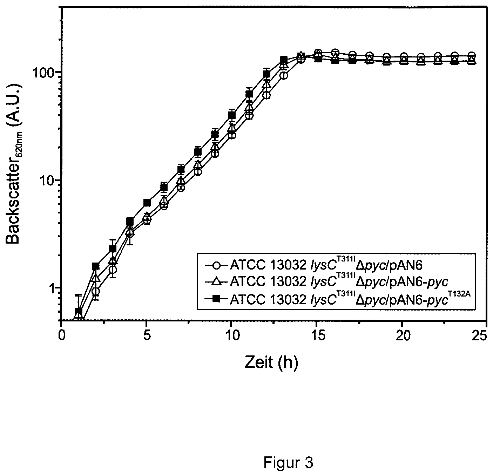

In Figur 3 ist das Wachstum des Stammes C. *glutamicum* ATCC 13032 *lys*C^{T311I} Δ*pyc* mit dem Plasmid pAN6-*pyc*^{T132A} dargestellt. In der Figur zeigt wiederum die Abszisse die Zeit in Stunden (h) und die Ordinate den Wert für Backscatter bei 620 nm (A.U.) Zur Kontrolle wurden die Stämme C. *glutamicum* ATCC 13032 *lys*C^{T311I} Δ*pyc*, welche entweder das Leerplasmid pAN6 oder das Plasmid pAN6-pyc mit nativer *pyc* tragen, mitgeführt. Alle Stämme wurden in CGXII Minimalmedium mit 4% (wt/vol) Glucose und 25 mg L⁻¹ Kanamycin in einem BioLector^{®} System bei 30 °C und 1200 rpm für 24 h kultiviert. Zu Beginn der Kultivierung wurde die Expression von pyc mit 1 mM IPTG (Isopropyl-β-D-thiogalactosid) induziert.

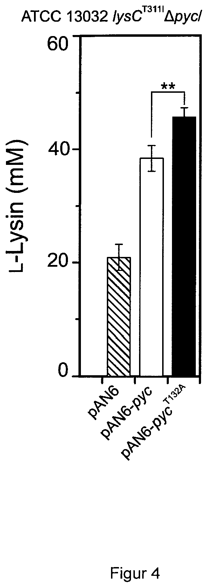

Figur 4 zeigt die L-Lysin-Produktion des Stammes C. *glutamicum* ATCC 13032 *lys*C^{T311I} Δ*pyc* mit dem Plasmid pAN6-*pyc*^{T132A}. Darin bezeichnet die Abszisse den Stamm *C. glutamicum* ATCC 13032 *lysC*^{T311I} Δ*pyc* enthaltend den Leervektor pAN6 (schraffierter Balken), den Vektor pAN6-pyc mit nativer Pyruvatcarboxylase (weißer Balken) oder den Vektor pAN6-*pyc*^{T132A} (schwarzer Balken) im Vergleich. Nach Kultivierung der Zellen in einem BioLector^{®} System für 24 h (s. Fig. 3), wurden die Zellen geerntet und die L-Lysin Konzentration im Überstand der jeweiligen Kulturen via reversed-phase HPLC mit ortho-Phthaldialdehyd-Derivatisierung bestimmt. Als Vergleich wurde die Lysin-Produktion im Stamm C. *glutamicum* ATCC 13032 *lys*C^{T311I} Δ*pyc*/pAN6 (ohne Pyc) und ATCC 13032 *lys*C^{T311I}/pAN6-*pyc* (mit nativer Pyc) gemessen. Die vorliegenden Daten stellen Mittelwerte und Standardabweichungen aus mindestens sechs biologischen Replikaten dar. Die gemessenen Lysin-Konzentrationen der einzelnen Mutanten wurden mittels t-Test verglichen. Dabei stehen die in der Abbildung gezeigten Sternchen für p ≤0.01. Es ist eine deutliche Erhöhung der finalen L-Lysin-Konzentration um 19% in dem Stamm mit der Pyc-T132A-Variante im Vergleich zu dem Stamm mit nativer Pyc zu erkennen.

Im Rahmen der Erfindung konnte eine Mutation im pyc-Gen identifiziert werden, die zu einer erhöhten L-Lysin-Produktion führt. Es wurde zunächst mittels error prone-PCR eine Plasmid-basierte Pyc-Mutanten-Bibliothek erstellt, die anschließend im Stamm ATCC1303 *lys*C^{T311I} Δ*pyc* mit Hilfe eines genetisch kodierten Lysin-Sensors (pSenLys) und Fluoreszenz-aktivierter Zellsortierung (FACS) zunächst auf erhöhte Fluoreszenz gescreent wurde. Die isolierten Zellen wurden anschließend vermehrt und auf erhöhte Lysinbildung getestet. Diese Methoden sind dem Fachmann bekannt (vgl. Binder et al., Genome Biol. 2012, 13:R40). Die dabei isolierten Gen- und Enzymvarianten wurden genetisch charakterisiert, was schließlich zur Identifizierung der erfindungsgemäßen Pyc-Variante führte, welche die Produktion von L-Lysin, sowie anderen, von Oxalacetat abgeleiteten Metaboliten, steigert. Die gefundene Mutation lautet T132A.

### 1. Chromosomal kodierte Pyc-Variante Pyc-T132A:

Messung der L-Lysin-Produktion von C. *glutamicum* ATCC 13032 *lys*C^{T311I} mit der chromosomal kodierten Pyc-Variante PycT132A:
Der Austausch des Threonin-Codons 132 im chromosomalen pyc-Gen durch ein Alanin-Codon erfolgte mittels zweifacher homologer Rekombination unter Verwendung des Vektors pK19mobsacB nach dem Fachmann bekannten Verfahren (Schäfer et al. 1994 Gene 145:69-73; Hochheim et al. 2017 Biotechnol Lett 39:283-288). Verglichen wurde die Produktion mit dem Stamm *C. glutamicum* ATCC 13032 *lys*C^{T311I} mit nativem chromosomalem pyc-Gen. Die Stämme wurden in 800 µl CGXII-Minimalmedium mit 4% (wt/vol) Glucose für 24 h in einem Biolector-System bei 30°C und 1200 rpm kultiviert (Fig.1). Die Start-OD bei 600 nm betrug 0,5. Nach 24 h wurden die Zellen der einzelnen Kulturen sedimentiert und die L-Lysin-Konzentration im Überstand gemessen. Die Messung erfolgte über reversed phase HPLC mit einer Vorsäulenderivatisierung der Aminosäuren über ortho-Phthaldialdehyd. Als mobile Phase wurde ein Gradient von 80% Lösung A (100 mM Natrium-Acetat (pH 7,2)) und 20% Lösung B (100% (vol/vol) Methanol) zu 20% Lösung A und 80% Lösung B verwendet. Das Beispiel zeigt, dass die untersuchte Pyc-Variante einen positiven Effekt auf die Lysin-Produktion hat und diese bis zu 7% gesteigert werden kann, wenn die Pyc-Variante Pyc-T132A anstelle des wildtypischen Pyc-Proteins chromosomal codiert ist.

### 2. Plasmid-kodierte Pyc-Variante Pyc-T132A:

Messung der L-Lysin-Produktion von *C. glutamicum* ATCC 13032 *lys*C^{T311I} Δ*pyc*/pAN6-*pyc*^{T132A}:
Verglichen wurde die Produktion mit dem Stamm *C. glutamicum* ATCC 13032 *lys*C^{T311I}Δ*pyc*/pAN6-*pyc* codierend die native Pyc und der Leerplasmidkontrolle C. *glutamicum* ATCC 13032 *lys*C^{T311I}Δ*pyc*/pAN6. Die Stämme wurden in 800 µl CGXII-Minimalmedium mit 4% (wt/vol) Glucose und 25 mg/l Kanamycin für 24 h in einem Biolector-System bei 30°C und 1200 rpm kultiviert (Fig.3). Die Start-OD bei 600 nm betrug 0,5. Nach 24 h wurden die Zellen der einzelnen Kulturen sedimentiert und die L-Lysin-Konzentration im Überstand gemessen. Die Messung erfolgte über reversed phase HPLC mit einer Vorsäulenderivatisierung der Aminosäuren über ortho-Phthaldialdehyd. Als mobile Phase wurde ein Gradient von 80% Lösung A (100 mM Natrium-Acetat (pH 7,2)) und 20% Lösung B (100% (vol/vol) Methanol) zu 20% Lösung A und 80% Lösung B verwendet. Das Beispiel zeigt, dass die Lysin-Produktion um bis zu 19% gesteigert werden kann, wenn die Pyc-Variante Pyc^{T132A} anstelle der wildtypischen Pyc auf dem Plasmid pAN6 codiert ist. (Fig.4)

Disclaimer:
Gegenstand der Erfindung sind nicht Mutationen, die aus nachstehendem Stand der Technik bekannt sind, wie Ohnishi et al. (Applied Microbiology and Biotechnology (2002) 58: 217-223), nämlich die chromosomale Einführung des Aminosäureaustausches Prolin zu Serin an Position 458 der Pyc von *C. glutamicum* in den *C. glutamicum-Stamm* AHD2, der auf dem Wildtyp ATCC 13032 basiert und zwei Punktmutationen trägt, Val59Ala im Gen für die Homoserin-Dehydrogenase (*hom*) und Thr311Ile im Gen für die Aspartatkinase (*lysC*) und aus der Schrift US 7,300,777 B2, mit einem Organismus, in welchem eine Pyruvatcarboxylase aus dem *C. glutamicum-Stamm* NRRL-B11474 mit mehreren Mutationen (M1V, E153D, A182S, A206S, H227R, A452G, D1120E) gegenüber der Pyc aus dem *C. glutamicum-*Stamm ATCC 21523 isoliert wurde.

## Patentansprüche

1. DNA-Sequenz kodierend für eine Pyruvatcarboxylase mit einer mindestens 85%igen Identität zu der Sequenz Nr.1, wobei das Triplett in Position 394-396 für Alanin kodiert.

2. DNA-Sequenz nach Anspruch 1,
**dadurch gekennzeichnet,**
**dass** sie eine DNA nach Sequenz Nr.1 ist.

3. Pyruvatcarboxylase mit einer mindestens 90%igen Identität zu der Pyruvatcarboxylase nach Sequenz Nr. 2, bei der sich in Position 132 Alanin befindet.

4. Pyruvatcarboxylase nach Anspruch3,
**dadurch gekennzeichnet,**
**dass** die Pyruvatcarboxylase eine Identität von mindestens 95% zu der Pyruvatcarboxylase nach Sequenz Nr. 2 besitzt.

5. Pyruvatcarboxylase nach Anspruch 3 oder 4,
**dadurch gekennzeichnet,**
**dass** sie eine Pyruvatcarboxylase nach Sequenz Nr. 2 ist.

6. Vektor,
**dadurch gekennzeichnet,**
**dass** er eine DNA-Sequenz nach einem der Ansprüche 1 oder 2 enthält.

7. Vektor nach Anspruch6,
**dadurch gekennzeichnet,**
**dass** er ein Plasmid ist.

8. Genetisch veränderter Mikroorganismus,
**dadurch gekennzeichnet,**
**dass** er eine DNA nach einem der Ansprüche 1 oder 2 enthält.

9. Genetisch veränderter Mikroorganismus nach Anspruch8,
**dadurch gekennzeichnet,**
**dass** er einen Vektor nach einem der Ansprüche 6 oder 7 enthält.

10. Genetisch veränderter Mikroorganismus nach Anspruch 8 oder9,
**dadurch gekennzeichnet,**
**dass** er ein Mikroorganismus aus der Gruppe bestehend aus den Gattungen *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* und *Clostridium* ist.

11. Verfahren zur Herstellung von Produkten, deren Biosynthese Oxalacetat als Vorstufe beinhaltet,
**dadurch gekennzeichnet,**
**dass** ein genetisch veränderter Mikroorganismus, enthaltend eine DNA nach den Ansprüchen 1 oder 2, der eine Pyruvatcarboxylase mit einer Sequenz nach einem der Ansprüche 3 bis 5 bildet, fermentiert wird, eine Anreicherung des Produktes im Medium oder in den Zellen durchgeführt wird.

12. Verfahren nach Anspruch 11,
**dadurch gekennzeichnet,**
**dass** das Produkt isoliert wird.

13. Verfahren nach Anspruch 11 oder 12,
**dadurch gekennzeichnet,**
**dass** als Mikroorganismus eine Spezies aus der Gruppe bestehend aus den Gattungen *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* und *Clostridium* eingesetzt wird.

14. Verfahren nach einem der Ansprüche 11 bis13,
**dadurch gekennzeichnet,**
**dass** eine Aminosäure der Aspartat-Familie hergestellt wird.

15. Verfahren nach Anspruch 14,
**dadurch gekennzeichnet,**
**dass** L-Lysin, L-Aspartat, L-Asparagin, L-Threonin, L-Isoleucin oder L-Methionin hergestellt wird.

16. Verfahren nach einem der Ansprüche 11 bis13,
**dadurch gekennzeichnet,**
**dass** Stoffe aus der Glutamat-Familie der Aminosäuren wie L-Glutamat, Glutamin L-Arginin, L-Prolin, L-Citrullin oder L-Ornithin, Intermediate des Citrat-Zyklus, wie Malat, Fumarat, Succinat, Malat, Fumarat Citrat, Isocitrat oder 2-Oxoglutarat, Diamine wie Diaminopentan oder Diaminobutan sowie weitere aus Oxalacetat hergestellte Stoffwechselprodukte, wie Itaconat, Ectoin, gamma-Aminobutyrat, Butanol, 1-Propanol, L-Citrullin, L-Ornithin, D-Arginin oder 4-Hydroxyprolin hergestellt werden.

17. Verfahren nach einem der Ansprüche 11 bis 16,
**dadurch gekennzeichnet,**
**dass** die Gene nach den Ansprüchen 1 oder 2 verstärkt exprimiert werden.

18. Genetisch verändertes Chromosom eines Mikroorganismus aus der Gruppe bestehend aus den Gattungen *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* und *Clostridium,* enthaltend eine DNA-Sequenz nach einem der Ansprüche 1 oder 2.

## Claims

1. A DNA sequence encoding a pyruvate carboxylase with at least 85% identity to the sequence No. 1, wherein the triplet, in position 394-396, encodes alanine.

2. A DNA sequence according to claim 1,
**characterised in**
**that** it is a DNA according to sequence No. 1.

3. A pyruvate carboxylase with at least 90% identity to the pyruvate carboxylase according to sequence No. 2, where alanine is located in position 132.

4. A pyruvate carboxylase according to claim 3,
**characterised in**
**that** the pyruvate carboxylase has an identity of at least 95% to the pyruvate carboxylase according to sequence No. 2.

5. A pyruvate carboxylase according to claim 3 or 4,
**characterised in**
**that** it is a pyruvate carboxylase according to sequence No. 2.

6. A vector,
**characterised in**
**that** it contains a DNA sequence according to any one of claims 1 or 2.

7. A vector according to claim 6,
**characterised in**
**that** it is a plasmid.

8. A genetically modified microorganism,
**characterised in**
**that** it contains a DNA according to any one of claims 1 or 2.

9. A genetically modified microorganism according to claim 8,
**characterised in**
**that** it contains a vector according to any one of claims 6 or 7.

10. A genetically modified microorganism according to claim 8 or 9,
**characterised in**
**that** it is a microorganism from the group consisting of the genera *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* and *Clostridium.*

11. A method for the manufacture of products, the biosynthesis of which includes oxalacetate as precursor,
**characterised in**
**that** a genetically modified microorganism, containing a DNA according to claims 1 or 2, which forms a pyruvate carboxylase having a sequence according to any one of claims 3 to 5, is fermented, an enrichment of the product is carried out in the medium or in the cells.

12. A method according to claim 11,
**characterised in**
**that** the product is isolated.

13. A method according to claim 11 or 12,
**characterised in**
**that** a species from the group consisting of the genera *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* and *Clostridium* is used as a microorganism.

14. A method according to any one of claims 11 to 13,
**characterised in**
**that** an amino acid of the aspartate family is manufactured.

15. A method according to claim 14,
**characterised in**
**that** L-lysine, L-aspartate, L-asparagine, L-threonine, L-isoleucine or L-methionine is manufactured.

16. A method according to any one of claims 11 to 13,
**characterised in**
**that** substances from the glutamate family of the amino acids such as L-glutamate, glutamine L-arginine, L-proline, L-citrulline or L-ornithine, intermediates of the citrate cycle, such as malate, fumarate, succinate, malate, fumarate citrate, isocitrate or 2-oxoglutarate, diamines such as diaminopentane or diaminobutane as well as additional metabolic products made from oxalacetate, such as itaconate, ectoine, gamma-aminobutyrate, butanol, 1-propanol, L-citrulline, L-ornithine, D-arginine or 4-hydroxyproline are manufactured.

17. A method according to any one of claims 11 to 16,
**characterised in**
**that** the genes according to claims 1 or 2 are increasingly expressed.

18. A genetically modified chromosome of a microorganism from the group consisting of the genera *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* and *Clostridium,* containing a DNA sequence according to any one of claims 1 or 2.

## Revendications

1. Séquence d'ADN codant pour une pyruvatecarboxylase ayant une identité d'au moins 85% avec la séquence n°1, dans laquelle le triplet en position 394 - 396 code pour l'alanine.

2. Séquence d'ADN suivant la revendication 1,
**caractérisée**
**en ce que** c'est un ADN suivant la séquence n°1.

3. Pyruvatecarboxylase ayant une identité d'au moins 90% avec la pyruvatecarboxylase suivant la séquence n°2, dans laquelle de l'alanine se trouve en position 132.

4. Pyruvatecarboxylase suivant la revendication 3,
**caractérisée**
**en ce que** la pyruvatecarboxylase possède une identité d'au moins 95% avec la pyruvatecarboxylase suivant la séquence n°2.

5. Pyruvate carboxylase suivant la revendication 3 ou 4,
**caractérisée**
**en ce que** c'est une pyruvatecarboxylase suivant la séquence n°2.

6. Vecteur,
**caractérisé**
**en ce qu'**il contient une séquence d'ADN suivant l'une des revendications 1 ou 2.

7. Vecteur suivant la revendication 6,
**caractérisé**
**en ce que** c'est un plasmide.

8. Micro-organisme modifié génétiquement,
**caractérisé**
**en ce qu'**il contient un ADN suivant l'une des revendications 1 ou 2.

9. Micro-organisme modifié génétiquement suivant la revendication 8,
**caractérisé**
**en ce qu'**il contient un vecteur suivant l'une des revendications 6 ou 7.

10. Micro-organisme modifié génétiquement suivant la revendication 8 ou 9,
**caractérisé**
**en ce que** c'est un micro-organisme du groupe constitué des genres *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio et Clostridium.*

11. Procédé de production de produit, dont la biosynthèse contient de l'oxalacétate comme précurseur,
**caractérisé**
**en ce que** l'on fait fermenter un micro-organisme modifié génétiquement, contenant un ADN suivant les revendications 1 ou 2, qui forme une pyruvatecarboxylase ayant une séquence suivant l'une des revendications 3 à 5, on effectue un enrichissement du produit dans le milieu ou dans les cellules.

12. Procédé suivant la revendication 11,
**caractérisé**
**en ce que** l'on isole le produit.

13. Procédé suivant la revendication 11 ou 12,
**caractérisé**
**en ce que** l'on utilise, comme micro-organisme, une espèce du groupe constituée des genres *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio* et *Clostridium.*

14. Procédé suivant l'une des revendications 11 à 13,
**caractérisé**
**en ce que** l'on produit un acide aminé de la famille de l'aspartate.

15. Procédé suivant la revendication 14,
**caractérisé**
**en ce que** l'on produit de la L-lysine, du L-aspartate, de la L-asparagine, de la L-thréonine, de la L'isoleucine ou de la L-méthionine.

16. Procédé suivant l'une des revendications 11 à 13,
**caractérisé**
**en ce que** l'on produit des substances de la famille du glutamate des acides aminés, comme du L-glutamate, de la glutamine-L-arginine, de la L-proline, de la L-citrine, ou de la L'ornithine, des intermédiaires du cycle citrate, comme malate, fumarate, succinate, malate, fumarate citrate, isocitrate ou 2-oxoglutarate, des diamines, comme le diaminopentane ou le diaminobutane, ainsi que d'autre produits du métabolisme produit à partir de l'oxalacétate, comme un itaconate, de l'éctoïne, un gamma-aminobutyrate, du butanol, du L-propanal, de la L-citruline, de la L-ornithine, de la D-arginine ou de la 4-hydroxyproline.

17. Procédé suivant l'une des revendications 11 à 16,
**caractérisé**
**en ce que** l'on exprime de manière amplifiée les gènes suivant les revendications 1 ou 2.

18. Chromosome modifié génétiquement d'un micro-organisme du groupe constitué des genres *Corynebacterium, Brevibacterium, Bacillus, Lactobacillus, Lactococcus, Candida, Pichia, Kluyveromyces, Saccharomyces, Escherichia, Zymomonas, Yarrowia, Methylobacterium, Ralstonia, Vibrio et Clostridium,* contenant une séquence d'ADN suivant l'une des revendications 1 ou 2.
